# EUROPEAN PATENT SPECIFICATION

(11) **EP 2 793 857 B1**
(45) Date of publication and mention of the grant of the patent: **13.06.2018**
(21) Application number: 12806098.5
(22) Date of filing: 19.12.2012
(51) Int. Cl.: A61K 9/08, A61K 47/10

(54) **DRUG DELIVERY TECHNOLOGY**
WIRKSTOFFFREISETZUNGSVERFAHREN
TECHNOLOGIE D'ADMINISTRATION DE MÉDICAMENT

(30) Priority: 21.12.2011 GB 201122041
(43) Date of publication of application: 29.10.2014
(73) Proprietor: LondonPharma Ltd., London EC3A 7AR (GB)
(72) Inventor: SAMS, Martin James, London EC3A 7AR (GB); HIGH, Juliet Victoria, London EC3A 7AR (GB); JAMIESON, Paul Andrew, London EC3A 7AR (GB); BOOLES, Clive, London EC3A 7AR (GB)
(74) Representative: Harris, Oliver John Richard
(86) International application number: PCT/GB2012/053184
(87) International publication number: WO 2013/093456

(56) References cited:
- WO-A1-02/05820
- WO-A1-99/66933
- WO-A1-2005/077374
- WO-A1-2007/002125
- WO-A2-2011/156405
- DE-A1-102005 009 240
- US-A1- 2011 263 606

## Description

### Field of the Invention

The invention relates to methods of delivery of PDE5 inhibitor sildenafil mesylate.

### Background

The development of drug delivery routes remains an important element in the progress of the pharmaceutical sciences. Once an active compound has been identified, the design of delivery mechanisms must overcome challenges of transporting the medicament to the required site of action in the body whilst addressing issues including shelf life, bioavailability, toxicity, and patient compliance. All of these challenges must be overcome to achieve the desired therapeutic effect. Amongst the drug delivery options, oral administration is by far the most common route, with other options including injection, inhalation,topical or transmucosal administration.

The oral delivery route faces perhaps the most challenging route for a pharmaceutical to reach the final site of action: the composition is prone to loss from the mouth or stomach (e.g. by spitting or vomiting); the composition must survive the acidic and enzymatically-active environment of the stomach; if not absorbed in the stomach, the medicament must survive the action of bile salts and further intestinal and bacterial enzymatic action within the intestinal tract, be able to cross from the lumen of the gut to the intestinal wall for absorption, and then survive the degradation processes of the liver following transport by the hepatic portal system, often resulting in poor availability due to the first pass effect. In addition, some patients can't take (or don't like taking) tablets, a common form for oral dosing. Despite these challenges, the oral route of drug administration remains the most common.

PDE5 inhibitors are inhibitors of type 5 cGMP-phosphodiesterase (PDE5).PDE5degrades cGMP in specific tissues such as the smooth muscle lining blood vessels supplying the corpus cavernosum of the penis and lining blood vessels within the lungs. PDE5 inhibitors are thus used to treat erectile dysfunction and pulmonary arterial hypertension (PAH). To date, PDE5 inhibitors have been administered exclusively orally, and a number of side-effect issues have arisen. Firstly, oral delivery provides quite variable levels of absorption for a particular PDE5 inhibitor dose, both intra-subject (possibly dependent on e.g. gastric content) (especially for sildenafil) and inter-subject. Secondly, a number of adverse effects have been reported, such asheadache, dizziness, flushing, gastrointestinal disturbances(such as indigestion, diarrhoea and vomiting) and visual disturbances (in the extreme, resulting in loss of vision). Use of nitrates with PDE5 inhibitors can cause hypotension (nitrates are used for treatment of angina).Thirdly, it takes a relatively long time for clinically useful concentrations of the PDE5 inhibitor to be absorbed into the blood post oral administration (a particular problem when treating erectile dysfunction).

It is among the objectives of the present invention to attempt a solution to these problems.

### Summary of the Invention

The inventors have surprisingly identified formulation conditions via which pharmaceutical compositions comprising a PDE5 inhibitor can be successfullydelivered to subjects by the sublingual transmucosal route, thereby avoidingat least the unwanted gastrointestinal adverse effects that arise with oral delivery (and possibly also other adverse effects e.g. visual disturbances). Sublingual transmucosal delivery also appears to provide more consistent absorption (intra- and inter-subject) than oral delivery. Furthermore, such sublingual transmucosal delivery is beneficial over the oral route in which the PDE5 inhibitor may be lost from the mouth orstomach (e.g. spitting or vomiting) or in which taking tablets is difficult or not possible.In addition, sublingual transmucosal delivery provides a more rapid progression to clinically useful concentrations of PDE5 inhibitor in the blood, providing a faster-acting product that should have particular benefits in the treatment of erectile dysfunction. The inventors have thus enabled the use of a PDE5 inhibitor for inhibiting PDE5 activity *in vivo,* where in said use said composition is delivered by the sublingual transmucosal route.

Accordingly, the invention provides a pharmaceutical composition for the transmucosal delivery of a PDE5 inhibitor, said composition comprising a PDE5 inhibitor and a carrier in which said PDE5 inhibitor is soluble or forms a suspension or emulsion. In particular the invention provides a pharmaceutical composition for use in a method of preventing or treating a sexual dysfunction, pulmonary hypertension, a vascular or cardiac or neurodegenerative condition, a physical injury, multiple sclerosis or fetal growth restriction in an individual, wherein the composition is delivered by sublingual administration as a spray, and wherein said composition comprises:
a PDE5 inhibitor; and
a carrier in which said PDE5 inhibitor is soluble or forms a suspension or emulsion.

In an aspect of the invention said PDE5 inhibitoris hydrophilic, preferablysildenafil mesylate, or a salt of vardenafil, said carrier comprises an aqueous solution, and said PDE5 inhibitor is solubilised in said carrier.

In an alternative aspect of the invention said PDE5 inhibitor is hydrophobic, preferably sildenafil, sildenafil citrate, vardenafil, or tadalafil or a salt thereof,said carrier comprises an aqueous solution, and said PDE5 inhibitor is suspended or emulsified in said carrier.

In a further aspect of the invention, said PDE5 inhibitor is lipophobic, preferably sildenafil, vardenafil or tadalafil, or a salt thereof, said carrier comprises an oil that comprises a glyceride, and said PDE5 inhibitor is suspended or emulsified in said carrier.

Where the carrier comprises an oil that comprises a glyceride, said oil preferably comprises a triglyceride. Preferably, said oil comprises a medium chain triglyceride (Ph Eur), preferably a miglyol, preferably miglyol 810, miglyol 812, miglyol 818, miglyol 829 or miglyol 840.

In preferred embodiments of any aspect of the invention, the composition further comprises a preservative and/oran antioxidant.

In preferred embodiments of any aspect of the invention, the composition further comprises a flavouring agent and/or a sweetener.

Where the carrier of the composition comprises an aqueous solution, preferably said flavouring agent comprises(i) a water-soluble flavouring agent and/or(ii) a lipophilic flavouring agent that is solubilised by means of a water-miscible oil-based solvent, and/or preferably said sweetener is water-soluble. Preferably said lipophilic flavouring agent comprises menthol, peppermint oil, lemon oil or aniseed oil and/or said water-miscible oil-based solvent comprises macrogolglycerol ricinoleate (Ph Eur). Preferably, said sweetener comprises sucralose.

Alternatively, where the carrier of the composition comprises an oil that comprises a glyceride, the composition preferably comprises a lipophilic flavouring agent, preferably menthol, vanillin or an essential oil (preferably orange oil, lemon oil, clove oil, peppermint oil, spearmint oil or aniseed oil).

Also provided is a composition according to the invention that is comprised within a container that comprises a delivery device, preferably wherein said delivery device dispenses the composition as a spray.

Further provided is a pharmaceutical composition comprising a PDE5 inhibitor for use in inhibiting PDE5 activity *in vivo,* where in said use said composition is delivered by the transmucosal route, and a composition according to the invention for use in inhibitingPDE5 activity *in vivo,* where in said use said composition is delivered by the transmucosal route, preferably wherein said use said composition is for use in treating a disease or condition responsive to a PDE5 inhibitor (such as erectile dysfunction or pulmonary arterial hypertension).

### Brief Description of the Drawing

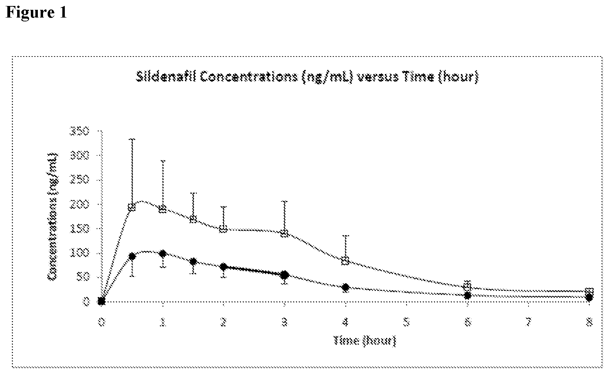

Figure 1 shows an absorption profile following sublingual delivery of sildenafil using a composition of the invention at25mg dose (solid circles) and 50mg dose (open squares).

### Detailed Description of the Invention

All references to transmucosal delivery/administration herein are preferably by sublingual delivery by spray.

The invention relates to a pharmaceutical composition for the transmucosal delivery of PDE5 inhibitor. sildenafil mesylate A PDE5 inhibitor is any inhibitor (e.g. competitive) of type 5 cGMP-phosphodiesterase (PDE5). Particular PDE5 inhibitors that are envisaged are the well-known PDE5 inhibitors sildenafil,vardenafil and tadalafil, and salts thereof. All of these medicaments have well-defined chemical structures that are widely published in drug reference sources (e.g. Martindale).

The composition comprises a carrier in which the PDE5 inhibitor is soluble or forms a suspension or emulsion, preferably wherein more than 50% of the PDE5 is solubilised or suspended/emulsified (respectively) in said carrier, preferably wherein at least 75% of the PDE5inhibitor is solubilised or suspended/emulsified, more preferably at least 85%, more preferably at least 90%, most preferably at least 99% (and preferably at a temperature between 5°C and 40°C). Preferably, the carrier is liquid at between 5°C and 40°C, and particularly is it preferred that the carrier is liquid at between 15°C and 40°C.

Preferably, thePDE5 inhibitor ispresent in the carrier at a concentration providing a required dosein a volume of no more than 1000microlitres of composition, more preferably in a volume of no more than 500microlitres, more preferably in a volume of no more than 200microlitres of composition, and most preferably in a volume of no more than 100microlitres of composition. In preferred embodiments the PDE5 inhibitor is present in said composition at a concentration of at least 5mg/ml, preferably at least 10mg/ml, more preferably at least 25mg/ml, more preferably at least 50mg/ml(and optionally at least 80mg/ml or at least 100mg/ml).

A further preferred feature is that the PDE5 inhibitor is stable in the composition, both with respect to physicochemical aspects such as remaining in solution or suspension/emulsion (as required) and in terms of chemical (including biochemical) degradation of the PDE5 inhibitor over time. It is particularly preferred, therefore, that the PDE5 inhibitor is stable within the composition, to pharmaceutically-acceptable limits, over a period of at least one month, preferably at least 2 months, more preferably at least 3 months, more preferably at least 6 months, more preferably at least 12 months, more preferably at least 18 months, more preferably at least 2 years, more preferably at least 3 years, more preferably at least 4 years, and most preferably at least 5 years, whilst kept at a temperature(s) between 4°C and 40°C and under the Relative Humidity conditions defined in the relevant ICH guidelines (preferably up to 25°C or 30°C).

In one embodiment the period of time from preparation of the composition to its administration is at least one month, at least 2 months, at least 6 months, at least 12 months, at least 18 months, at least 2 years or at least 3 years.

Note that the terms 'hydrophilic', 'lipophilic', 'hydrophobic' and 'lipophobic' as used herein refer to the intrinsic chemical properties of the described entity.

### Compositions solubilisinghydrophilic PDE5 inhibitors

In an aspect of the invention the PDE5 inhibitor ishydrophilic, preferablysildenafil mesylate, or a salt of vardenafil, and the PDE5 inhibitor is solubilised in a carrier that comprises (or consists essentially of) an aqueous solution (including water itself).

In preferred embodiments the PDE5 inhibitor is sildenafil mesylate. Sildenafil is typically dosed orally at 25-100mg. Therefore, in this aspect of the composition of the invention, sildenafil mesylate is preferably provided at a concentration providing a concentration of sildenafil of at least 5mg/ml, more preferably at least 50mg/ml (enabling e.g. a dose of 25mg to be delivered with a 500µl actuation), more preferably at least 100mg/ml (enabling e.g. a dose of 25mg to be delivered with a 250µl actuation). It is particularly preferred that such a composition comprising sildenafil mesylate further comprisesa flavouring agent and/or a sweetener (see below).

In alternative embodiments the PDE5 inhibitor is a salt of vardenafil (preferably vardenafil hydrochloride trihydrate). Vardenafil is typically dosed orally at 10-20mg. Therefore, in this aspect of the composition of the invention, the vardenafil salt is preferably provided at a concentration providing a concentration of vardenafil of at least 5mg/ml, more preferably at least 50mg/ml (enabling e.g. a dose of 25mg to be delivered with a 500µl actuation). It is particularly preferred that such a composition comprising a vardenafil salt further comprises a flavouring agent and/or a sweetener (see below).

It is surprising to find that such essentially aqueous compositions have been used to successfully deliver PDE5 inhibitors by the transmucosal route given the expected propensity for such hydrophilic compositions to be washed down into the gastrointestinal tract.

### Co-solvents

In embodiments of the above compositions of the invention said composition further comprises a co-solvent i.e. any agent that increases the solubility of the PDE5 inhibitor in the carrier. Where the PDE5 inhibitor is sildenafil mesylate the co-solvent preferably does not comprisediethylene glycol monoethyl ether, glycerol or propylene glycol. A co-solvent can be used at between 1% and 49% w/w or v/v, more preferably between 1% and 40%, most preferably between 5% and 35%.

Note that diethylene glycol monoethyl ether is defined in the European Pharmacopoeia Monograph 1198 as: 2-(2-Ethoxyethoxy) ethanol, produced by condensation of ethylene oxide and alcohol, followed by distillation.

### Suspensions/Emulsions

In other aspects of the invention the composition of the invention comprises a PDE5 inhibitor anda carrier in which said PDE5 inhibitor forms a suspension or emulsion.

In particular embodiments the PDE5 inhibitor is hydrophobic, preferably sildenafil, sildenafil citrate, vardenafil, or tadalafil, or a salt thereof, and the PDE5 inhibitor is suspended or emulsified in a carrier that comprises (or consists essentially of) an aqueous solution (including water itself).

In alternative embodiments the PDE5 inhibitor is lipophobic, preferably sildenafil, vardenafil or tadalafil, or a salt thereof, and the PDE5 inhibitor is suspendedor emulsified in a carrier that comprises (or consists essentially of) an oil that comprises (or consists essentially of) a glyceride.

### Carriers comprising an oil that comprises a glyceride

Such carriers might comprise (or consists essentially of) an oil comprising a monoglyceride, a diglyceride and/or (and preferably) a triglyceride. Preferred oils includeGlycerol mono-oleates (Ph Eur);Fractionated Palm Kernel Oil (Ph Eur);Hydrogenated Cottonseed Oil (Ph Eur);Omega-3-Marine Triglycerides (Ph Eur);Fish Oil, Rich in Omega-3-Acids (Ph Eur);Cod Liver Oil (Ph Eur).

Preferred oils that comprise (or consist essentially of) a triglyceride includeRefined Maize Oil (Ph Eur),Virgin Castor Oil (Ph Eur),Refined Olive Oil (Ph Eur),Refined Rapeseed Oil (Ph Eur), Vegetable Fatty Oils (Ph Eur) and, in particular, Medium Chain Triglycerides (Ph Eur).

Medium chain length triglycerides are defined in the European Pharmacopoeia Monograph 0868, as:
A mixture of triglycerides of saturated fatty acids, mainly of caprylic acid (octanoic acid, C₈H₁₆O₂) and of capric acid (decanoic acid, C₁₀H₂₀O₂). Medium-chain triglycerides are obtained from the oil extracted from the hard, dried fraction of the endosperm of *Cocos nucifera* L. or from the dried endosperm of *Elaeis guineensis* Jacq. When Medium-chain Triglycerides are prepared from the endosperm of *Cocos nucifera* L., the title Fractionated Coconut Oil may be used.Medium chain length triglycerides have a minimum 95.0 per cent of saturated fatty acids with 8 and 10 carbon atoms. Further chemical and physical properties are described in the European Pharmacopoeia Monograph 0868, and equivalent documents.

In preferred embodiments the oil comprises a miglyol, preferably miglyol 810, miglyol 812, miglyol 818, miglyol 829 ormiglyol 840.

### Further optional components

In preferred embodiments of any of said compositions, the composition further comprises a preservative (i.e. any agent that kills or inhibits the growth of a microorganism), such asethanol or benzalkonium chloride. Potassium sorbate is suitable where the PDE5 inhibitor is vardenafil, but the preservative preferably does not comprise potassium sorbate or methyl parahydroxybenzoate (or a sodium salt thereof) if the PDE5 inhibitor is sildenafil mesylate.In preferred embodiments the composition alternatively or additionally comprises an antioxidant. Preferably, said preservative and/or said antioxidant is present at an effective concentration, preferably at a concentration of at least 0.1% w/w or v/v, preferably at least 0.5%, and preferably at a concentration within published safety limits.

In preferred embodiments of any of said compositions, the composition further comprisesa flavouring and/or a sweetener.

In an aspect of the invention, wherein the carrier of the composition comprises an aqueous solution, the compositioncomprises a flavouring agent comprising(or consisting essentially of) a water-soluble flavouring agentand/or a lipophilic flavouring agent that is solubilised by means of a water-miscible oil-based solvent. Such a composition might alternatively or additionally comprise a water-soluble sweetener.In preferred embodiments the lipophilic flavouring agent comprises (or consists essentially of) menthol, peppermint oil, lemon oil or aniseed oil - it has surprisingly been found that these particular flavourings can be solubilised in an aqueous solution when using a water-miscible oil-based solvent. The flavouring agent is preferably present at a concentration of at least 0.1% w/w, preferably at least 0.5% and optionally up to 2%. The water-miscible oil-based solvent is preferably present at a concentration of at least 1% w/w, preferably at least 5% and optionally up to 10% or up to 25%. A particularly effective water-miscible oil-based solvent comprises (or consists essentially of) macrogolglycerol ricinoleate (Ph Eur).Preferably such a solvent is not used above 17% w/w. Macrogolglycerol Ricinoleate is defined in the European Pharmacopoeia Monograph 1082, as:
Contains mainly ricinoleyl glycerol ethoxylated with 30 to 50 molecules of ethylene oxide (nominal value), with small amounts of macrogol ricinoleate and of the corresponding free glycols. It results from the reaction of castor oil with ethylene oxide.

The water-soluble sweetenerpreferably comprises (or consists essentially of) sucralose, and is preferably present at a concentration of at least 0.05% w/w, preferably at least 0.1%. Where the PDE5 inhibitor is sildenafil mesylate the water-soluble sweetener preferably does not comprise glycerol, saccharin or sodium saccharin.

Particularly good taste improvement was achieved using a combination of a flavouring agent (preferably a fruit or mint flavour and/or lipophilic [solubilised by means of a water-miscible oil-based solvent]) and a sweetener (preferably water-soluble, preferably sucralose).

In an alternative aspect of the invention, wherein the carrier of the composition comprises an oil that comprises a glyceride, the composition comprises a lipophilic flavouring agent, such as menthol, vanillin or an essential oil (e.g. orange oil, lemon oil, clove oil, peppermint oil, spearmint oil, or aniseed oil), preferably at a concentration of at least 0.1% v/v, preferably at least 0.5%.

In one embodiment the composition may also comprise a selective serotonin reuptake inhibitor (SSRI), such as dapoxetine. Such compositions are preferably used to prevent premature ejaculation. The composition may comprise another agent (which is not an SSRI) which prevents or treats premature ejaculation.

### Potentially excluded components

There might be reasons to optionally exclude ethanol, for example where the composition is to be used in cultural or religious contexts where ethanol intake is not permitted.Hence in some preferred embodiments the composition is substantially free of ethanol e.g. comprises <1%w/w or v/v ethanol, more preferably less than 0.5% ethanol, and most preferably less than 0.1% ethanol.

Furthermore, it is possible to exclude propellants as these can be irritating to the mucosa. In optional embodiments, therefore, the composition of the invention is substantially free of propellant e.g. comprises <1%w/w or v/v propellant, more preferably less than 0.5% propellant, and most preferably less than 0.1% propellant.

### Delivery Devices

Preferably the compositions of the present invention are comprised within a container that comprises a delivery device; the device can dispense the composition as a multiple discharge or, preferably, as a single discharge (in order to minimise the risk of a serious side effect i.e. there is a risk of overdose if too many actuations are taken from a multiple dose unit). Preferably the device is non-pressurised.

The compositions of the present invention can be delivered as a liquid bolus or, preferably, as a spray. Preferably, said spray comprisesliquid droplets having a mean diameter of at least about 10 microns, preferably at least 20 microns, more preferably from about 20 to about 200 microns, most preferably from about 20 to about 100 microns. Preferably the compositions are delivered as liquid droplets that have a size distribution of from about 5 microns to about 500 microns, preferably from about 10 microns to about 200 microns, more preferably from about 20 microns to about 100 microns. Choice of these (larger) droplet sizes means that the droplets have larger weight and this is preferable in the invention because a larger weight increases the chances that the droplet, and therefore the PDE5 inhibitor, falls rapidly onto the mucosa thereby reducing the possibility that the droplets become entrained in breath and expelled from the mouth, or taken into the lungs.

It is particularly preferred that each individual or successive dose has a volume of less than 1000 microlitres. The use of small dose volumes reduces the likelihood that the composition will be swallowed, or spat out, by the patient. The likelihood is reduced further by use of smaller volumes (especially in the paediatric context) and so in further preferred embodiments, each dose has a volume of less than 600 microlitres; less than 500 microlitres; less than 400 microlitres; less than 300 microlitres; less than 200 microlitres; or even less than 100 microlitres. Smaller volumes are especially preferred for paediatric use.

Preferably, the delivery device according to these aspects comprises a spray device, preferably a non-pressurised spray device, and especially a pump spray device. The use of a pump spray device increases the area of mucosa to which the composition is applied, thereby increasing absorption and minimising the likelihood that the medicament is swallowed.

### Outcomes

The invention also provides a pharmaceutical composition comprising a PDE5 inhibitor(such as any of the compositions of the present invention) for use in inhibiting PDE5 activity *in vivo,* where in said use said composition is delivered by the transmucosal route (such as via the nasal, buccal or sublingual route, preferably by the sublingual route). By inhibiting PDE5 activity, PDE5 inhibitorsact to treat a disease or condition responsive to a PDE5 inhibitor, such as erectile dysfunction, pulmonary arterial hypertension or any condition mentioned herein; and the invention thus provides said composition for any of these specific effects.

The invention thereby also provides a method of treating a human or animal (preferably mammal) subject in need of aPDE5 inhibitor, comprising the administration to saidsubject of a therapeutically effective amount of a composition comprising a PDE5 inhibitor (such as a composition of the present invention), whereby administration is by the transmucosal route (such as via the nasal, buccal or sublingual route, preferably by the sublingual route).The invention thus also provides such a method for the treatment of a disease or condition responsive to a PDE5 inhibitor, such as erectile dysfunction, pulmonary arterial hypertension or any condition mentioned herein.

### Advantageous Pharmacokinetics

The advantageous pharmacokinetics discovered by the inventors means that administration according to the invention is particularly suited to certain patient groups. Thus in preferred embodiments the invention relates to treating patients for whom it is important to avoid variability in dosage and/or who are susceptible to certain conditions or side effects.

The invention concerns prevention or treatment of male or female sexual dysfunction, pulmonary hypertension, a vascular or cardiac or neurodegenerative condition, a physical injury, multiple sclerosis or fetal growth restriction. In particular the invention concerns prevention or treatment of erectile dysfunction, premature ejaculation, ischaemia/reperfusion injury, myocardial infarction, vascular endothelial dysfunction, cardiac hypertrophy, heart failure, cardiomyopathy, Raynauds, cerebro vascular disease, Alzheimers, drug induced sexual dysfunction (preferably antidepressant induced sexual dysfunction), testis injury after torsion/detorsion or female sexual dysfunction.

Preferred individuals to be treated include those that cannot, or are unwilling to, swallow tablets. The individual may have a gastrointestinal condition, for example dysphagia (difficulty in swallowing) or may suffer from nausea and/or heartburn when given tablets. The patient may have a gastrointestinal obstruction, such as in the upper part of the gastrointestinal tract. The patient may be a child (10 years old or less, such as 5 years old or less, or 3 years old or less), and thus be less able to take a tablet. The patient may have a preference for a sublingual spray or may have reduced consciousness or be unconscious.

In another embodiment the individual is not in hospital and/or is not under the care of a medical practitioner. The individual may thus be self-administering the composition. The advantageous pharmacokinetics means the invention is particularly suited for treating sexual dysfunctions, such as erectile dysfunction, where administration can be done shortly before sexual activity. In one embodiment administration is less than 2 hours, preferably less than 1 hour before sexual activity.

For embodiments relating to treating erectile dysfunction, the treatment is preferably of an individual unable to achieve or maintain a penile erection sufficient for satisfactory sexual performance. In such embodiments the individual is preferably at least 50 years old, such as at least 60 or at least 70 years old.

In one embodiment the individual is on long term PDE5 inhibitor therapy, for example has had at least one administration of a PDE5 inhibitor every 15 or 30 days for more than 200, 300 or 500 days.

In one embodiment the individual is given 2, 3, 4, 5 or 6 to 10 administrations according to the invention, and all of these administrations have an effective dosage which differs from the first administration by less than 15% of the effective dosage of the first administration.

In a preferred embodiment the Tₘₐₓ (time to maximal plasma concentration) is less than 2 hours from administration, preferably less than 1 hour or less than 0.5 hours. In one embodiment the time to an effective concentration in the blood is less than 2 hours from administration, preferably less than 1 hour or less than 0.5 hours

### Examples

### Example 1 - Sildenafil formulation summary

Neither sildenafil (base) nor sildenafil citrate were found to be soluble in Miglyol 810(medium-chain triglycerides Ph Eur)or water at a useful concentration and addition of ethanol did not improve solubility. However, a mesylate salt of sildenafil was found to be soluble in aqueous formulations, up to 100mg/ml.

A GMP batch (11-089) was made with sildenafilmesylate provided at 75mg/ml(62.5mg/ml base):

### Sildenafil 6.25mg (base) per 0.1ml actuation (Batch size 2000ml)

| Material | Weight (g) |
|---|---|
| Sildenafil mesylate | 150.0 |
| Ethanol, Anhydrous Ph Eur | 400.0 |
| Purified water Ph Eur | To vol |

Solubility and stability were acceptable after at least 2 months (5°C and 25°C). In a continuation of these experiments solubility and stability were found to be acceptable after at least 6 months at 5°C, 25°, 30°C and 40°C at ICH relative humidity conditions. This batch was tested in the clinic (see later section).

It was found that sildenafil mesylate was soluble up to 100mg/ml in water and that this was unaltered with the use of 20% w/w ethanol (though ethanol can also act as a preservative). The use of alternative co-solvents (20% w/w) reduced solubility, to 80mg/ml (propylene glycol) or 60mg/ml (Transcutol HP [diethylene glycol monoethyl ether Ph Eur] or glycerol). The inclusion of caffeine and sodium dihydrogen phosphate did not appear to significantly improve the solubility of the sildenafil mesylate.In the absence of ethanol an alternative preservative could be used, thoughmethyl parabens and potassium sorbate precipitated out of solution.

Sildenafil mesylate solutionshave a midly unpleasant taste; this was sufficiently masked with strawberry flavour at 0. 1%and sucralose at 0.05%, though it was found that both saccharin and sodium saccharin are probably unsuitable as sweeteners.

### Example 2 - Vardenafil formulation summary

Vardenafil (hydrochloride trihydrate) was found to be insoluble in Miglyol 810 (with or without ethanol) but was readily soluble in water, up to at least 50mg/ml. Potassium sorbate (0.2%) was found to be suitable as a preservative. Suitable sweeteners were sucralose (0.05%) and suitable flavourings were strawberry flavour 501094 (0.5%) and orange juice flavour 506304 (0.5%).

### Example 3 - Further formulation work for taste improvement

Particularly good improvements in taste were obtained using lipophilic flavours, once conditions had been identified in which select examples could be dissolved within the aqueous carrier. Cremophor EL (macrogolglycerol ricinoleate Ph Eur) was identified as a component that was able to maintain menthol, peppermint oil, aniseed and lemon oil in solution. These flavours were firstly mixed at 10%w/wwith Cremophor EL, and the resultant combination thenmixed with water (10%w/w in purified water). Conversely, vanillin, orange oil and clove oil (after combination with Cremophor EL) did not remain miscible when mixed with water. Note that vanillin also degraded in Cremophor EL, rendering it unusable. Note that Cremophor EL did not mix well with water above ∼17% and should therefore not be used above this concentration. The action of Cremophor EL here is that of a water-miscible oil-based solvent. Sildenafil and vardenafil remained in solution at a concentration of at least 60mg/ml and 30mg/ml, respectively, in the presence of 10% Cremophor EL.

### Example 4 - Tadalafil formulation summary

Tadalafil(base) was found to be insoluble inwater, Miglyol 810, and a number of other potential solvents e.g. PEG (even using ethanol as a co-solvent).

### Example 5 - Pharmacokinetic studies

A confidential phase 1, single centre, open label, randomised crossover trial was carried out to evaluate the rate and extent of absorption of a sublingual formulation of sildenafil mesylate in healthy male subjects.

### Objectives

The primary objectives of this study were to:
- assess the sublingual absorption of the study medication
- establish dose proportionality between different doses administered

The secondary objectives of this study were to:
- establish the safety, tolerability, local tolerance and taste acceptance of the study medication

### Methodology/study design

Subjects were required to provide their written informed consent prior to any study related procedures being conducted. Subjects were screened for eligibility within 28 days of first study admission on Day -1. Eligible subjects were required to participate in two study treatment periods, each at a different dose level with a washout period of at least 24 hours between the study periods. For each treatment period, subjects were admitted to the clinical research unit (CRU) the evening prior to dosing. Subjects received their treatments in a randomised way. Subjects were closely monitored in the clinic for at least 8 hours after dosing. After the last treatment period, subjects returned for a post study follow up visit.

### Number of Subjects

A total of 8 healthy male subjects were enrolled in the study.

### Number of Subjects Analysed

All 8 subjects entered and completed the study and were included in the safety and pharmacokinetic populations.

### Diagnosis and Main Criteria for Inclusion

Healthy male subjects aged 18-55 of any ethnic origin, with a body mass index (BMI) within the range 18 - 25, and a minimum weight of at least 55 kg were eligible for the trial. Subjects had to be in good health as determined by a medical history, medical examination, electrocardiogram, laboratory tests, and serology. Subjects had to be willing to complete all required assessments. Prior to any study procedures, subjects were required to understand as well as sign the informed consent form (ICF).

### Test Product, Dose and Mode of Administration Batch Number(s)

Sildenafil mesylate sublingual spray 6.25mg (base) per 100µl actuation, dosed at 25mg or 50mg; batch number 11-089.

### Duration of Treatment

The duration of the study was approximately 6 weeks for each subject including the screening and follow-up period. Subjects had 2 inpatient treatment periods with a washout period of at least 24 hours between treatments.

### Criteria for Evaluation

### Pharmacokinetic variables -

Blood samples were collected for pharmacokinetic (PK) analysis at the following time points:predose (within 60 mins), and 0.5, 1, 1.5, 2, 3, 4, 6 and 8 hours after dosing.

The following pharmacokinetic parameters were calculated by standard non-compartmental methods for the traditional two stage analysis using WinNonlin Ver 5.0.1: AUC ₀₋ₜ, AUC _{0-∞}, Cₘₐₓ, Tₘₐₓ, λ_{z}, t _{1/2}, CL/F, and V/F. SPSS Ver 17.0 was used for the statistical analysis.

### Safety variables -

Safety was evaluated by adverse event (AE) monitoring, evaluation of any medically significant changes, based on physical examination findings, vital signs, and electrocardiograms (ECGs) assessments, clinical laboratory testing, local tolerance and taste acceptance.

### Statistical Methods

All statistical analyses were appropriate to the nature and distribution of the data collected. These are detailed in the pharmacokinetic analysis plan (PAP).

### Pharmacokinetics

Pharmacokinetic samples from all treatment periods were analysed for concentrations of the active ingredients only. Statistical analysis was based on data from all treatment periods for all subjects studied. Individual subject profiles and mean profiles of the plasma concentration for each active by treatment were produced.

The PK parameters AUC₀₋ₜ, AUC_{0-∞}, Cₘₐₓ, Tₘₐₓ, λ_{z}, t_{1/2}, CL/F, and V/F were listed by treatment for each subject, and where appropriate suitable statistical comparisons were made. Descriptive summaries including n, arithmetic mean, SD, CV (%), geometric mean, 95% CI for the arithmetic mean, median, minimum and maximum were presented for each dose level. Tₘₐₓ was summarised using n, median, minimum and maximum values.

### Safety Analyses

Safety data was summarised by descriptive methods; continuous variables by summary statistics and categorical data by absolute and relative frequencies.

### Summary of Pharmacokinetic Results and Conclusions

Figure 1 shows the resultant absorption profile (a plot of mean plasma concentration versus time, with standard deviation) following sublingual delivery of sildenafil at 25mg dose (solid circles) and 50mg dose (open squares), with outlying sample excluded.

Summary PK Parameters for sildenafil sublingual spray by dose level -

| **Pharmacokinetic Parameter** | **Summary Statistic** | **Sildenafil** | | |
|---|---|---|---|---|
| | | **Dose Level 1 (25 mg) (outlying sample excluded)** | **Dose Level 1 (25 mg) (all samples)** | **Dose Level 2 (50 mg)** |
| AUC₀₋₈ (ng.h/mL) | Mean | 319.47 | 349.51 | 714.14 |
| | CV% | 29 | 39 | 30 |
| AUC_{0-∞} (ng.h/mL) | Mean | 343.69 | 373.73 | 761.89 |
| | CV% | 29 | 37 | 30 |
| Cₘₐₓ (ng/mL) | Mean | 109.09 | 176.25 | 250.30 |
| | CV% | 31 | 120 | 40 |
| t_{½} (hours) | Mean | 1.85 | 1.85 | 1.69 |
| | CV% | 24 | 24 | 19 |
| tₘₐₓ (hours) | Median | 0.94 | 1.00 | 1.31 |
| | Range | 0.50 - 1.50 | 27 | 0.5 - 3.0 |
| Vz/F (L) | Mean | 226.51 | 217.93 | 184.20 |
| | CV% | 70 | 75 | 53 |
| CL/F (L/hour) | Mean | 82.06 | 78.80 | 79.30 |
| | CV% | 47 | 52 | 68 |

Summary of statistical analysis of bioavailability and dose proportionality-

| **Pharmacokinetic Parameter** | **Summary Statistic** | **Sildenafil (all samples)** | **Sildenafil (outlying sample excluded)** |
|---|---|---|---|
| Log ₁₀ AUC₀₋₈ | Ratio of means | 2.065 | 2.203 |
| | 90% Cl | 1.660-2.560 | 1.895 - 2.562 |
| Log ₁₀ Cₘₐₓ | Ratio of means | 1.841 | 2.218 |
| | 90% Cl | 1.237-2.740 | 1.873 - 2.626 |
| Tₘₐₓ^{a} | Z | -0.11 | -0.41 |
| | P value | 0.91 | 0.68 |
| t_{½} | T | 0.94 | N/A |
| | P value | 0.38 | |

### Discussion

Sildenafil is rapidly and extensively absorbed following sublingual administration. There was no significant difference between the Tₘₐₓ for either treatment, being 0.94 h and 1.31h following 25 mg and 50 mg sildenafil sublingual spray respectively. For the 25 mg and 50 mg formulations respectively, 85% and 78% of the peak concentration was reached within the first 30 minutes after dosing; this means that clinically useful concentrations of sildenafil were obtained in the blood by 30 minutes after dosing, which compares very favourably with the ∼1hr delay seen with oral delivery. For the 25 mg treatment, one subject had an anomalous plasma concentration at the 1 h time point that was more than 6 times the Cₘₐₓ. Excluding the outlying time point, the ratio of the means for AUC was 2.203 (90% CI: 1.895-2.562), indicating that the 50 mg dose was slightly higher than dose proportional. Likewise, the ratio of the means for Cₘₐₓ was 2.218 (90% CI: 1.873-2.626), also indicative of the 50 mg dose being slightly higher than dose proportional. Regarding the reported values of t_{½}, which were similar for both treatments, it appears from the above figure that the terminal elimination phase had not been adequately characterised. For most of the PK parameters, the inter-individual variability as expressed by CV% was reasonably low, being ≤ 30% in most instances (with the outlying sample excluded).

Overall, both doses of sildenafil sublingual spray were found to be safe and well tolerated. One subject reported AEs related to local tolerability but these were mild and resolved spontaneously without any intervention by the end of the study. Another subject reported throat irritation that may have been related to the study medication. No other subject experienced local events (apart from taste). There were very few AEs reported generally (8 related and 1 unrelated) and all were of mild severity and resolved spontaneously without any intervention. There were no clinically significant changes in vital signs, ECG parameters, physical examinations or clinical laboratory evaluations. The taste acceptance was generally good, although there were reports of very unpleasant taste after receiving a single administration of 25 mg and 50 mg sildenafil sublingual spray at the early time points but at the later time points, no taste was reported by any of the subjects. It can be concluded that both 25 mg sildenafil sublingual spray and 50 mg sublingual spray were safe and well tolerated in healthy male subjects.

### Stability Testing

Samples of spray were stored for the required times at the ICH conditions shown in the table below. The sildenafil and any degradant were assayed using a stability indicating HPLC method. The results show very good stability under the conditions tested (5 to 40°C) after 6 months storage. This would indicate the formulation would have a suitably long shelf life once confirmed by stability studies conducted over a longer timeframe.

### Sildenafil pilot batch stability data

| | | **Content (mg/ml)** | **Degradation (%)** |
|---|---|---|---|
| **Initial** | | 74.5 | No peaks > 0.1 |
| | | | |
| **5°C** | **2 weeks** | 74.2 | No peaks > 0.1 |
| | **4 weeks** | 75.9 | No peaks > 0.1 |
| | **6 weeks** | 75.2 | No peaks > 0.1 |
| | **12 weeks** | 75.1 | No peaks > 0.1 |
| | **24 weeks** | 77.6 | No peaks > 0.1 |
| | | | |
| **25°C/60%RH** | **2 weeks** | 75.4 | No peaks > 0.1 |
| | **6 weeks** | 75.1 | No peaks > 0.1 |
| | **12 weeks** | 75.7 | No peaks > 0.1 |
| | **24 weeks** | 77.0 | No peaks > 0.1 |
| | | | |
| **30°C/65%RH** | **12 weeks** | 76.4 | No peaks > 0.1 |
| | **24 weeks** | 74.9 | No peaks > 0.1 |
| | | | |
| **40°C/75%RH** | **12 weeks** | 75.9 | No peaks > 0.1 |
| | **24 weeks** | 75.3 | No peaks > 0.1 |

### Sildenafil GMP batch (11-089) stability data

| | | **Content (mg/ml)** | **Degradation (%)** |
|---|---|---|---|
| | | | |
| **Initial** | | 78.6 | No peaks > 0.1 |
| | | | |
| **5°C** | **2 weeks** | 74.7 | No peaks > 0.1 |
| | **4 weeks** | 76.9 | No peaks > 0.1 |
| | **12 weeks** | 77.0 | No peaks > 0.1 |
| | **24 weeks** | 76.5 | No peaks > 0.1 |
| | | | |
| **25°C/60%RH** | **2 weeks** | 74.9 | No peaks > 0.1 |
| | **4 weeks** | 77.0 | No peaks > 0.1 |
| | **12 weeks** | 76.6 | No peaks > 0.1 |
| | **24 weeks** | 77.2 | No peaks > 0.1 |
| | | | |
| **30°C/65%RH** | **12 weeks** | 76.5 | No peaks > 0.1 |
| | **24 weeks** | 76.3 | No peaks > 0.1 |
| | | | |
| **40°C/75%RH** | **12 weeks** | 76.9 | No peaks > 0.1 |
| | **24 weeks** | 77.9 | No peaks > 0.1 |

## Claims

1. A pharmaceutical composition for use in a method of preventing or treating a sexual dysfunction, pulmonary hypertension, a vascular or cardiac or neurodegenerative condition, a physical injury, multiple sclerosis or fetal growth restriction in an individual, wherein the composition is delivered by sublingual administration as a spray, and wherein said composition comprises sildenafil mesylate and a carrier that comprises an aqueous solution, wherein said sildenafil mesylate is solubilised in said carrier.

2. A composition for use according to claim 1, further comprising a preservative and/or an antioxidant.

3. A composition for use according to any preceding claim, wherein the composition does not comprise propylene glycol.

4. A composition for use according to any preceding claim, further comprising a flavouring agent and/or a sweetener.

5. A composition for use according to claim 4 wherein:
(a) said flavouring agent comprises:
(i) a water-soluble flavouring agent; and/or
(ii) a lipophilic flavouring agent that is solubilised by means of a water-miscible oil-based solvent; and/or
(b) said sweetener is water-soluble.

6. A composition for use according to claim 5 wherein:
(a). said lipophilic flavouring agent comprises menthol, peppermint oil, lemon oil or aniseed oil;
(b). said water-miscible oil-based solvent comprises macrogolglycerol ricinoleate (Ph Eur); and/or
(c). said sweetener comprises sucralose.

7. A composition for use according to any one of the preceding claims wherein the individual:
- cannot, or is unwilling to, ingest tablets, or
- has a gastrointestinal condition or gastrointestinal obstruction, or
- has reduced consciousness, or
- is 10 years old or less, such as 5 years old or less, or 3 years old or less.

8. A composition for use according to any one of the preceding claims wherein:
- multiple administrations are given and there is a low level of variability in absorption of the composition by the individual for the different administrations, or
- the Tₘₐₓ is less than 2 hours, preferably less than 1 hour or less than 0.5 hours, or
- the period of time between preparation of the composition and administration to the individual is at least 6 months, or
- the composition is administered for prevention or treatment of erectile dysfunction, premature ejaculation, ischaemia/reperfusion injury, myocardial infarction, vascular endothelial dysfunction, cardiac hypertrophy, heart failure, cardiomyopathy, Raynauds, cerebro vascular disease, Alzheimers, drug induced sexual dysfunction (preferably antidepressant induced sexual dysfunction), testis injury after torsion/detorsion or female sexual dysfunction.

## Patentansprüche

1. Pharmazeutische Zusammensetzung zur Verwendung in einem Verfahren zur Vorbeugung oder Behandlung einer sexuellen Dysfunktion, pulmonaler Hypertonie, eines vaskulären oder kardialen oder neurodegenerativen Zustands, einer körperlichen Verletzung, einer Multiplen Sklerose oder einer fetalen Wachstumsretardierung bei einer Person, wobei die Zusammensetzung durch sublinguale Verabreichung als Spray abgegeben wird, und wobei die Zusammensetzung Sildenafilmesylat und einen Träger umfasst, der eine wässrige Lösung umfasst, wobei das Sildenafilmesylat in dem Träger solubilisiert ist.

2. Zusammensetzung zur Verwendung nach Anspruch 1, die ferner ein Konservierungsmittel und/oder ein Antioxidans umfasst.

3. Zusammensetzung zur Verwendung nach einem der vorstehenden Ansprüche, wobei die Zusammensetzung kein Propylenglykol umfasst.

4. Zusammensetzung zur Verwendung nach einem der vorstehenden Ansprüche, die ferner einen Aromastoff und/oder einen Süßstoff umfasst.

5. Zusammensetzung zur Verwendung nach Anspruch 4, wobei:
(a) der Aromastoff Folgendes enthält:
(i) einen wasserlöslichen Aromastoff; und/oder
(ii) einen lipophilen Aromastoff, der vermittels eines wassermischbaren Lösungsmittels auf Ölbasis solubilisiert ist; und/oder
(b) den Süßstoff, der wasserlöslich ist.

6. Zusammensetzung zur Verwendung nach Anspruch 5, wobei:
(a). der lipophile Aromastoff Menthol, Pfefferminzöl, Zitronenöl oder Anisöl enthält;
(b). das wassermischbare Lösungsmittel auf Ölbasis Makrogolglycerin-Ricinoleat (Ph Eur) enthält; und/oder
(c). der Süßstoff Sucralose enthält.

7. Zusammensetzung zur Verwendung nach einem der vorstehenden Ansprüche, wobei die Person:
- keine Tabletten einnehmen kann oder will, oder
- eine gastrointestinale Erkrankung oder eine gastrointestinale Obstruktion hat, oder
- Bewusstseinsstörungen hat, oder
- 10 Jahre oder jünger ist, z. B. 5 Jahre oder jünger, oder 3 Jahre oder jünger ist.

8. Zusammensetzung zur Verwendung nach einem der vorstehenden Ansprüche, wobei:
- mehrere Verabreichungen erfolgen und eine geringe Variabilität in der Absorption der Zusammensetzung durch die Person bei den einzelnen Verabreichungen besteht, oder
- die Tₘₐₓ weniger als 2 Stunden beträgt, vorzugsweise weniger als 1 Stunde oder weniger als 0,5 Stunden, oder
- der Zeitraum zwischen der Zubereitung der Zusammensetzung und der Verabreichung an die Person mindestens 6 Monate beträgt, oder
- die Zusammensetzung zur Vorbeugung oder Behandlung von erektiler Dysfunktion, vorzeitiger Ejakulation, Ischämie/Reperfusionsverletzung, Myokardinfarkt, vaskulärer endothelialer Dysfunktion, Herzhypertrophie, Herzinsuffizienz, Kardiomyopathie, Raynauds, zerebrale Gefäßerkrankungen, Alzheimer, drogeninduzierter sexueller Dysfunktion (vorzugsweise durch Antidepressiva induzierter sexueller Dysfunktion), Hodenverletzung nach Torsion/Detorsion oder weiblicher sexueller Dysfunktion verabreicht wird.

## Revendications

1. Composition pharmaceutique destinée à être utilisée dans une méthode de prévention ou de traitement d'un dysfonctionnement sexuel, de l'hypertension pulmonaire, d'un état vasculaire ou cardiaque ou neurodégénératif, d'une blessure physique, de la sclérose en plaques ou d'un retard de croissance foetale chez un individu, dans laquelle la composition est délivrée par administration par voie sublinguale sous la forme d'un spray, et dans laquelle ladite composition comprend du mésylate de sildénafil et un véhicule qui comprend une solution aqueuse, dans laquelle ledit mésylate de sildénafil est solubilisé dans ledit véhicule.

2. Composition destinée à être utilisée selon la revendication 1, comprenant en outre un conservateur et/ou un antioxydant.

3. Composition destinée à être utilisée selon l'une quelconque des revendications précédentes, dans laquelle la composition ne comprend pas de propylèneglycol.

4. Composition destinée à être utilisée selon l'une quelconque des revendications précédentes, comprenant en outre un agent aromatisant et/ou un édulcorant.

5. Composition destinée à être utilisée selon la revendication 4, dans laquelle
(a) ledit agent aromatisant comprend :
(i) un agent aromatisant soluble dans l'eau ; et/ou
(ii) un agent aromatisant lipophile qui est solubilisé au moyen d'un solvant à base d'huile miscible avec l'eau ; et/ou
(b) ledit édulcorant est soluble dans l'eau.

6. Composition destinée à être utilisée selon la revendication 5, dans laquelle :
(a). ledit agent aromatisant lipophile comprend du menthol, de l'essence de menthe poivrée, de l'essence de citron ou de l'essence d'anis ;
(b). ledit solvant à base d'huile miscible avec l'eau comprend du ricinoléate de macrogol-glycérol (Ph Eur) ; et/ou
(c). ledit édulcorant comprend du sucralose.

7. Composition destinée à être utilisée selon l'une quelconque des revendications précédentes, dans laquelle l'individu :
- ne peut pas, ou ne souhaite pas, ingérer de comprimés, ou
- souffre d'un état gastro-intestinal ou d'une obstruction gastro-intestinale, ou
- présente un état de conscience réduit, ou
- est âgé de 10 ans ou moins, tel que 5 ans ou moins, ou 3 ans ou moins.

8. Composition destinée à être utilisée selon l'une quelconque des revendications précédentes, dans laquelle :
- des administrations multiples sont données et il y a un faible niveau de variabilité de l'absorption de la composition par l'individu pour les différentes administrations, ou
- la Tₘₐₓ est inférieure à 2 heures, de préférence inférieure à 1 heure ou inférieure à 0,5 heure, ou
- la période de temps entre la préparation de la composition et l'administration à l'individu est d'au moins 6 mois, ou
- la composition est administrée pour prévenir ou traiter un dysfonctionnement érectile, une éjaculation précoce, une lésion par ischémie/reperfusion, un infarctus du myocarde, un dysfonctionnement endothélial vasculaire, une hypertrophie cardiaque, une insuffisance cardiaque, une cardiomyopathie, la maladie de Raynaud, une maladie cérébro-vasculaire, la maladie d'Alzheimer, un dysfonctionnement sexuel induit par des substances médicamenteuses (de préférence un dysfonctionnement sexuel induit par un antidépresseur), une lésion des testicules après torsion/détorsion ou un dysfonctionnement sexuel féminin.
